# EUROPEAN PATENT SPECIFICATION

(11) **EP 0 779 059 B1**
(45) Date of publication and mention of the grant of the patent: **21.04.2004**
(21) Application number: 96203457.5
(22) Date of filing: 06.12.1996
(51) Int. Cl.: A61B 5/042

(54) **Catheter with plate-like electrode array**
Katheter mit plattenförmiger Elektrodenanordnung
Cathéter comportant un ensemble d'électrodes en forme de plaque

(30) Priority: 13.12.1995 NL 1001890
(43) Date of publication of application: 18.06.1997
(73) Proprietor: Cordis Corporation, Miami Lakes Florida 33014 (US)
(72) Inventor: Siekmayer, Gerd, 52525 Waldfeucht-Haaren (DE); van Erp, Wilhelmus Petrus Martinus Maria, 9351 KS Leek (NL)
(74) Representative: 't Jong, Bastiaan Jacob

(56) References cited:
- EP-A- 0 682 911
- WO-A-94/07411
- WO-A-94/21165
- US-A- 4 976 711
- US-A- 5 255 679

## Description

The invention relates to a catheter comprising a tube-like basic body with a proximal end and a distal end. In the usual manner a connecting member has been arranged at the proximal end. At the distal end the catheter carries an electrode array. In the state of use measurements of the electrical activity of the heart can for instance be taken with the electrodes of the electrode array. Such a catheter is known from WO 94/07411, which has been used as basis for the delineation of claim 1 in the two-part form.

In the case of a tachycardia for instance, it is desirable to form a clear picture of the electrical activity in certain sections of the internal wall of the heart. When this picture has been obtained, ablation can be carried out locally in order to remedy the tachycardia.

According to the invention a catheter is provided with which at one go a measurement can be taken over a large area and/or an ablation carried out.

The catheter can be introduced into the patient with the electrode array in folded state, so that it has a relatively small diameter. When the electrode array has been positioned in the target position, the carrier is unfolded, so that the electrode array can be operative over the entire surface area. According to the invention the sheet of memory metal can easily be brought into the unfolded and folded state, controlled from the proximal end, by activating the heating means via the supply line. With the heating means switched on, the carrier assumes the unfolded state and when the heating means are switched off, the carrier of memory metal is folded.

Preferably the measure as set out in claim 2 is employed. On introducing the catheter, the electrode array is kept in the retracted state in order to prevent traumata. Once it has arrived at the target position, the electrode array is extended and subsequently unfolded, so that it becomes operative.

A very suitable embodiment is additionally characterised in claim 3. Extending and retracting the electrode array can be effected in a properly controlled manner by moving the inner tube-like body in relation to the outer tube-like body at the proximal end of the catheter.

In order to restore the electrode array to the folded state, the measures as set out in claim 4 can be employed in a suitable manner. The guiding means can for instance be somewhat funnel-shaped, so that on retracting the electrode array a force working inwards is applied to it in order to bring about the folded state.

To ensure that the electrode array and the carrier have a relatively small diameter in the folded state, the measure as set out in claim 5 is preferably employed. As a result the electrode array can have a relatively large surface area. The width of the electrode array can measure a number of times the diameter of the catheter.

A suitable further development is characterised in claim 6. Selectively switching off the heating means will first fold a section of the carrier corresponding to one of the separate sections of the heating means and subsequently, one by one, successive sections, so that a programmed folding movement is achieved, which facilitates folding the carrier, together with the electrode array, in a reliable manner into a small diameter.

A further development of the catheter according to the invention is characterised in claim 7. The resilient, compressible cushion ensures that the electrode array can be placed evenly against, in particular, the wall of the heart. Even when, in the unfolded state, the carrier does not extent entirely parallel to this wall of the heart, a good contact between all electrodes and the wall of the heart is achieved after all.

A suitable embodiment is additionally characterised in claim 8. In the unfolded state the bag can be filled with fluid in order to achieve the desired resilient compressibility, and prior to folding the fluid is removed from the bag, so that in the folded state the electrode array will have a minimal cross-section.

Another suitable embodiment of the catheter according to the invention is characterised in claim 10. By making the balloon swell up, for instance by inflating it, the electrode array can be brought into contact with a surface to be treated.

In order to protect the balloon during the introduction of the catheter the measure as set out in claim 11 is preferably employed. In the retracted state of the balloon, the latter is stored away in a protected manner inside the basic body.

When the carrier comprises a balloon, the measure as set out in claim 12 is employed in a favourable manner. The shape of the balloon can be influenced by a varying filling pressure of the compartments. The compartment which is turned away from the electrode array can for instance be inflated hard, whereas the compartment on the side of the electrode array is kept much softer, as a result of which a good contact is obtained between the electrode array and the surface to be treated, whilst at the same time the entire device is sufficiently firm to guarantee a good contact.

According to a further development the measure as set out in claim 14 is employed. As a result a large number of electrodes can be employed over a relatively large surface area of the electrode array, without it resulting in a proportionally large number of signal lines extending through the basic body of the catheter. From the multiplexer only one signal line needs to run to the proximal end.

The multiplexer can be made in such a way that it can also transmit ablation energy from the proximal end to selected electrodes of the electrode array, in order to be able to carry out a programmed ablation pattern using the electrode array.

A reliable embodiment of the catheter according to the invention, in which case the electrode array has once again a minimal thickness in order to have a minimal diameter in the folded state, is achieved with the measure as set out in claim 15.

Additionally, the measure as set out in claim 16 is preferably employed. By arranging the connecting lines on the other side of the foil substrate than the electrodes, the electrodes can be arranged in a closely fitting manner, so that a very good measurement over the entire surface of the electrode array can be obtained and also, if the catheter has been fitted out for that purpose, an accurate ablation can be carried out over the entire surface of the electrode array.

The measure as set out in claim 17 is employed in a suitable manner in order to minimize the space occupied by the multiplexer.

To enable pushing the electrode array sufficiently firm against the tissue to be investigated and treated respectively, the measure as set out in claim 18 is preferably employed. As a result the distal end of the catheter will become very stable which is necessary to carry out the treatment.

The invention will be explained in greater detail in the following description with reference to the attached drawings.
- Figure 1: shows a partly broken away perspective view of the distal end of a catheter according to an embodiment of the invention.
- Figure 2: shows the distal end with the electrode array illustrated in figure 1 when being folded.
- Figure 3: shows a partly broken away longitudinal cross-section of the catheter with the electrode array in the retracted state.
- Figure 4: shows a partly broken away view of a catheter according to another embodiment of the invention.
- Figure 5: shows a cross-section along the line V-V of figure 4.
- Figure 6: shows yet another embodiment.
- Figure 7: shows a cross-section along the line VII-VII of figure 7.
- Figure 8: illustrates the way in which the carrier is wound up.
- Figure 9: shows an embodiment of the invention whereby the carrier comprises an inflatable balloon.
- Figure 10: shows the embodiment of figure 9 whereby the balloon has been retracted inside the basic body.
- Figure 11: illustrates a cross-section through a carrier comprising a balloon.

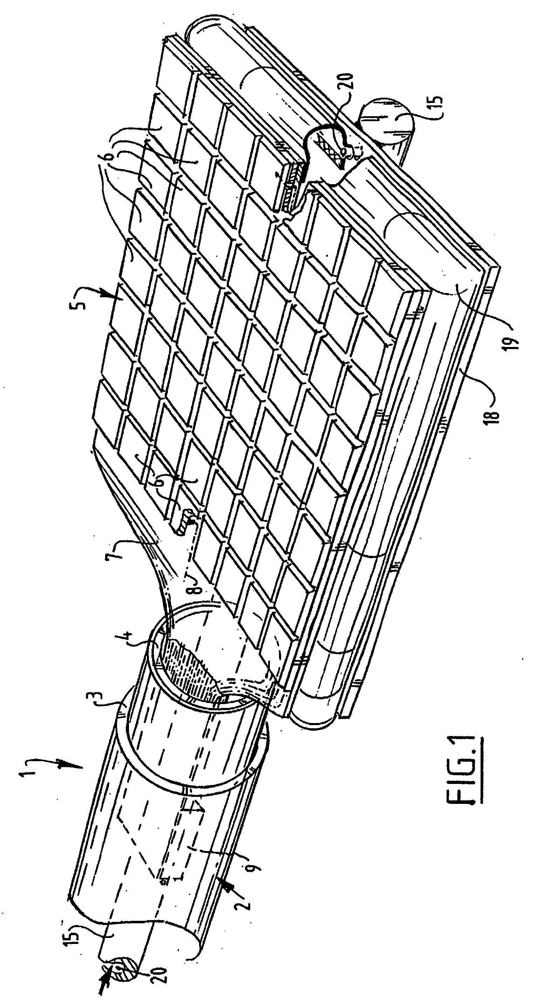

Of the catheter shown in the figures, only the distal end with the electrode array has been illustrated. The catheter 1 comprises in the usual manner a tube-like basic body 2 which extends from a proximal end, which remains outside the body of the patient when in use, to the distal end shown in figure 1.

With the embodiment shown, the basic body 2 comprises an outer tube-like element 3 with a central lumen inside of which an inner tube-like element 4 has been received, which is movable in a longitudinal direction.

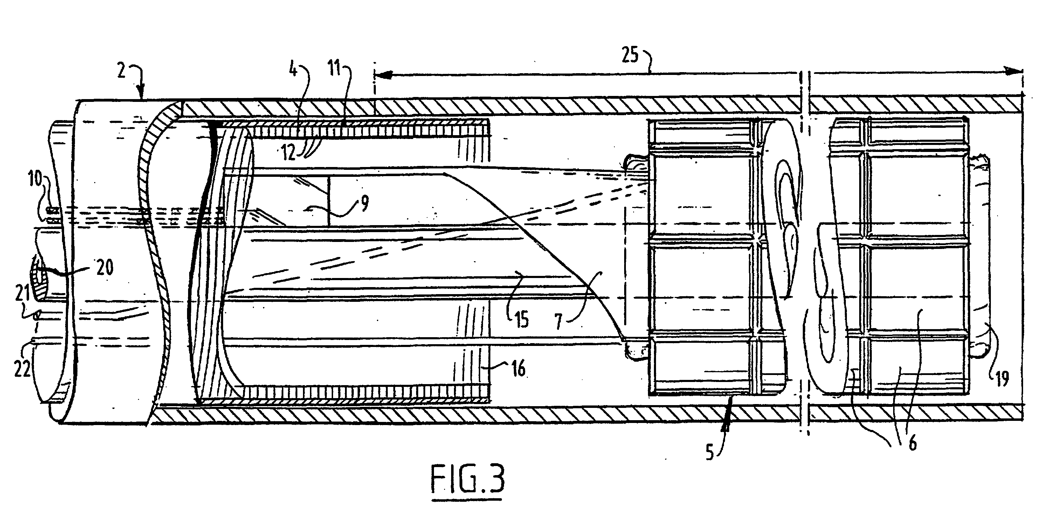

The inner tube-like element 4 is, as can be seen in figure 3, made up of a core 12 which has been formed by a helically coiled steel wire with a rectangular cross-section and is surrounded by a closely fitting outer sheath 11. By employing this construction, the thickness of the wall of the inner tube-like element 4 can be kept to a minimum, so that the overall thickness of the catheter can remain limited.

A bar-like support 15 has been fixed inside the end of the inner tube-like element, for instance by means of cement 16. As can be seen in figure 1, a plate-like carrier has been arranged on this support 15. On the carrier 18 a bag 19 has been mounted, which can be filled with a fluid via a channel 20 inside the support 15. Finally, the electrode array 5 has been arranged on top of the bag 19.

With this example of an embodiment the electrode array 5 is rectangular in shape and is made up of a great number of electrodes 6. The electrodes 6 have been made in the form of printed wiring on a foil substrate 7. As a result the electrode array 5 is pliable.

At the bottom of the foil substrate 7 not illustrated in figure 1, conductors have been arranged in the form of printed wiring which are connected, each time, with electrodes 6 via metallized openings in the foil 7. From each electrode 6 one conductor runs along the back of the foil substrate 7 to a multiplexer 9, which has been mounted on a narrower end-section of this foil. The electrode array has been fixed with this narrower section in the end of the inner tube-like element 4.

In figure 1, one of the lines which runs from an electrode 6 to the multiplexer 9 has been illustrated schematically and is indicated with the reference number 8.

From the multiplexer 9, signal lines 10 extend to the proximal end of the catheter 1.

In the state illustrated in figure 1, the catheter 1 can be used to map electrical activity in the inner wall of the heart of a patient. Especially in the case of tachyarrhythmias this is desirable in order to map pathways of undesired electrical activity. To this end the electrodes 6 of the electrode array 5 are manoeuvred against the wall of the heart. Because of the springy, compressible support of the bag 9 filled with fluid, the electrodes 6 can make proper contact with the surface of the wall.

After taking measurements and establishing undesired pathways, an ablation treatment can be carried out in order to disturb the undesired pathways. Via the signal lines 10 the electrodes 6, which are to ablate the wall of the heart against which they are positioned, are activated by means of the multiplexer 9. In this way the undesired pathways can be interrupted very selectively and at exactly the right place.

After the treatment, the electrode array 5 has to be folded again from the unfolded state illustrated in figure 1, in order to be able to remove it from the body of the patient.

For this purpose the carrier 18 of the electrode array 6 has been made of memory metal. The unfolded state of the carrier 18 illustrated in figure 1, that is to say the state in which it forms a more or less flat sheet, is the relatively relaxed state of the memory metal at raised temperature. The folded state, to be described in greater detail below, is the relatively relaxed state at body temperature. In order to bring the carrier 18 from the folded into the unfolded state, heating means have been arranged on the carrier 18, which can be turned on via lines 21, 22 which extend to the proximal end. When turned on, the carrier 18 is heated to above transition temperature, as a result of which the relatively relaxed unfolded state illustrated, is effected. The transition temperature can for instance be something like 45°C.

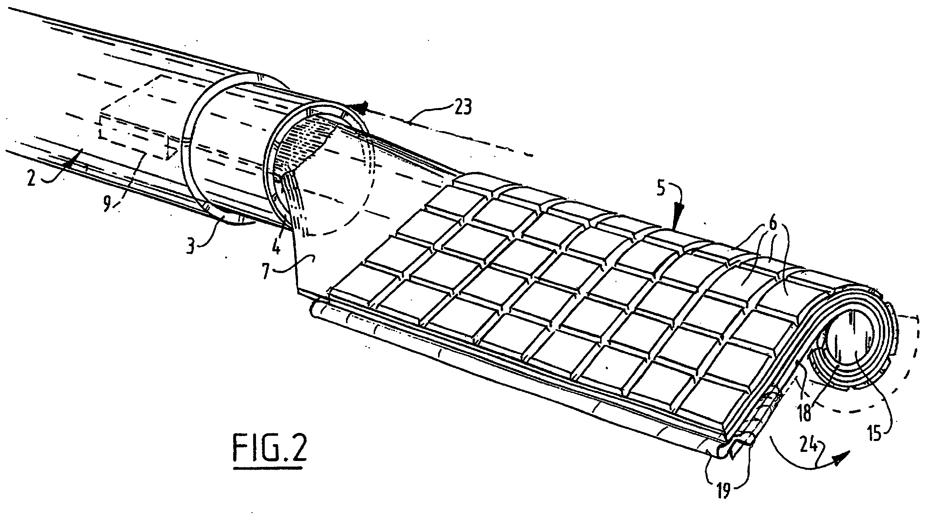

In order to fold the electrode array 5, the heating means are turned off. With the embodiment shown here, the heating means have been arranged in two separate sections, that is to say separated in the longitudinal direction of the catheter. Figure 2 illustrates the state when the back section of the heating means, as seen in the figures, is turned off. As a result the back section of the carrier 18 will cool down to body temperature and adopt the corresponding stable position, which corresponds to a form rolled up around the longitudinal axis.

In order to move the carrier into the folded state, the fluid has been removed from the bag 19, so as to obtain a minimal thickness of the assembly.

After the first section of the carrier has turned into the folded state of rest, the second section of the heating means is turned off, as a result of which also the front section of the carrier 18, as seen in figures 1 and 2, will roll up in the direction of the arrow 24 and resume the folded state. Next the electrode array 5 can be pushed into the outer tube-like body 2 by pulling the inner tube-like body 4, in relation to the outer tube-like body 2, outwards at the proximal end. Thus the state as illustrated schematically in figure 3 is brought about. The electrode array 5 has been moved inwards over a distance 25 in the direction indicated by the arrow 23 of figure 2, so that it is enclosed completely by the outer tube-like body 3.

It will be clear that also on introduction of the catheter into the patient, the electrode array 5 is kept in the folded and retracted state, until the distal end of the catheter has reached the target position, in particular the heart of the patient. Then the electrode array will be extended and the heating means activated as a result of which the electrode array will unfold into the state illustrated in figure 1 and will be ready for use.

With a somewhat altered embodiment of the catheter illustrated in figure 1, the support 15 can be made in such a manner that it can be retracted separately in relation to the inner tube-like element 4. The support 15 will in that case be fixed to the carrier 18 only close to the latter's most distal section. By moving, when in use, the support 15 in relation to the inner tube-like element 4 in the direction of the proximal end, the carrier 18, and consequently the electrode array 5, will be deformed into a convex shape, which provides an extra possibility to achieve proper contact of the electrode array 5 with for instance the wall of the heart.

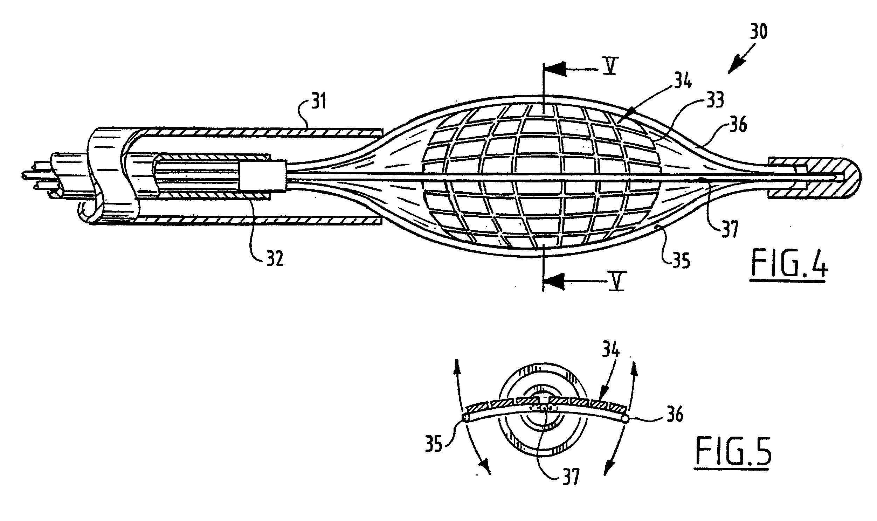

With the catheter 30 as shown in figure 4 the carrier comprises a pliable sheet such as a foil 33. The electrode array 34 has been arranged on this foil, for instance by means of a deposition technique.

Along two opposite sides, the sheet 33 is connected to wire like elements 35, 36. These wire like elements have been received in an inner tube-like element 32 of the basic body 31 and extend, via this tube-like element 32, to the proximal end of the catheter. In addition to these two wire like elements 35, 36 along the edges of the sheet 33, a central wire like element 37 has been arranged as well, which supports the sheet 33 in the centre.

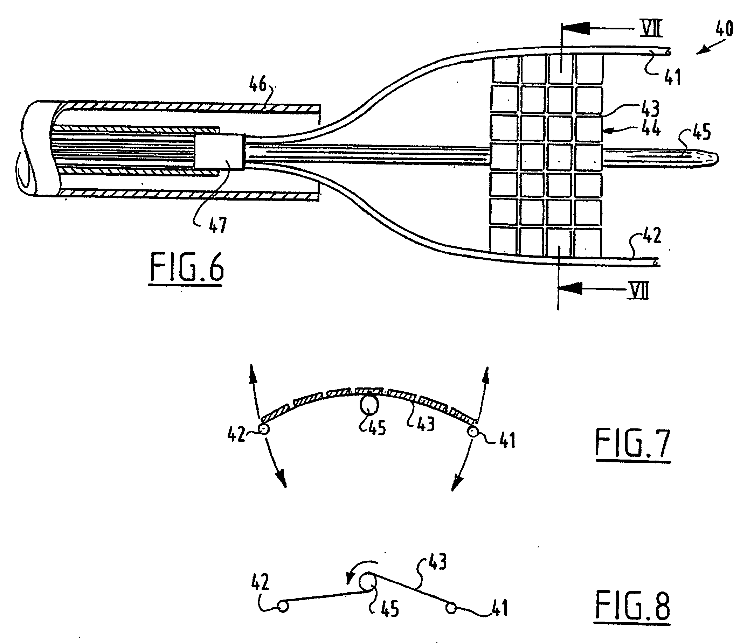

As can be seen in figure 5, the curve of the sheet 33 can be altered by rotating the wire like elements 35, 36 around their longitudinal axis. For this purpose these wire like elements 35, 36 have been provided with operating means at their ends protruding from the proximal end of the basic body 31. These have not been illustrated here.

The inner tube-like element 32 has been received in the basic body 31 in a movable manner, and by pulling at its proximal end the assembly of wire like elements 35, 36 bending outwards and the foil connected thereto, can be pulled into the basic body 31. The wire like elements 35, 36 are resilient so that they bend outwards automatically when extended and stretch the foil 33 by doing so.

The catheter illustrated in figures 4 and 5 can also be further developed in a suitable manner so that the sheet 33 and the electrode array 34 arranged to it can curve around an axis at right angles to the longitudinal direction of the catheter. To achieve this, the central wire like element 37 will be made so that it can be moved separately in a way analogous to the one described when referring to figure 1. By pulling at this element 37 at the proximal end of the catheter, the sheet 33 will curve, so that the sheet 33 can curve in two directions around two axes at right angles to one another. Suitable manipulation of the elements 35, 36, 37 can consequently ensure proper contact between the electrode array 34 and for instance the wall of a heart.

As shown in figure 6, the catheter 40 also comprises a carrier in the form of a foil 43 on top of which the electrode array 44 has been arranged. The carrier 43 is connected with opposite sides to wire like elements 41, 42 which are elastic and push the opposite sides of the sheet apart in a resilient manner, so that, in the unfolded state shown in figure 6, the carrier 43 is kept stretched.

The sheet 43 is also connected to a central pin 45 at a point in between the sides connected with the wire like elements 41, 42, which serves to support the sheet 43 and to fold the carrier in order to be able to retract it into the basic body 46.

As can be seen in figure 7, also in the case of this embodiment a suitable curve can be given to the sheet 43 carrying the electrode array 44, by rotating the wire like elements 41, 42.

The operative end of the catheter 40 has been received inside the basic body 46 when inserting the catheter. As soon as the distal end of the catheter 40 has arrived at the position where the treatment is to be carried out, this operative end-section is extended by moving the inner tube-like element 47 in a longitudinal direction in relation to the outer tube-like element 46.

Following treatment the operative end has to be received once again inside the tube-like basic body 46. To this end the central pin 45 is turned in the direction shown in figure 8, as a result of which the sheet 43 is wound around this pin and the wire like elements 41, 42 are pulled towards this pin 45. Thus the assembly is gathered to form a small diameter and can be received in the basic body 46 by pulling the inner tube-like element 47 inwards.

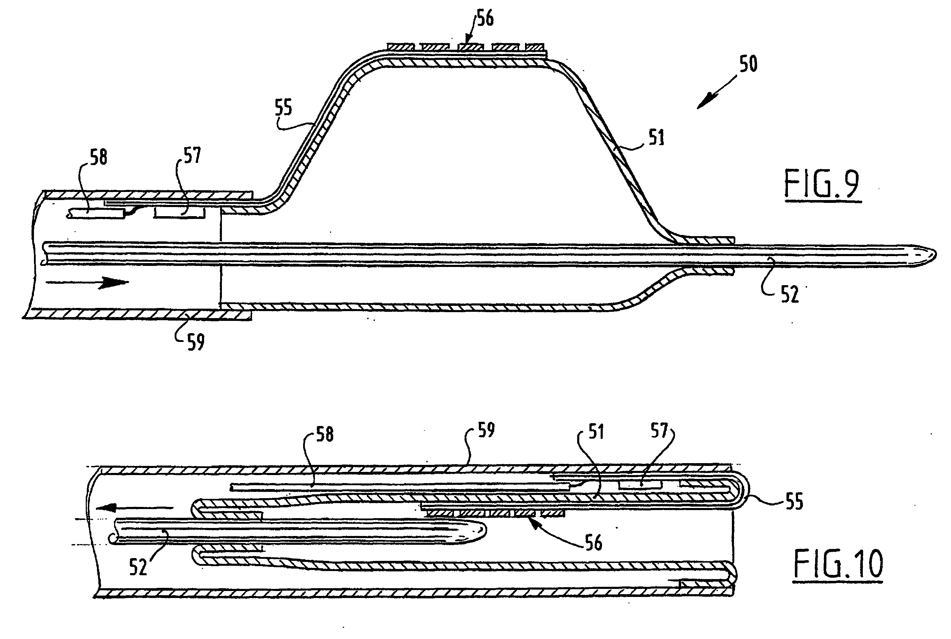

With the embodiment 50 of the figures 9 and 10 the carrier comprises an inflatable balloon 51. The latter may be preformed in a suitable manner to ensure that the electrode array 56 arranged on the wall thereof unfolds into a suitable shape as soon as the balloon is inflated.

In this case, the electrode array 56 is connected to a multiplexer 57 via lines 55 arranged on the outside of the balloon, so that only one single line 58 has to be led to the proximal end of the catheter.

Instead of using one single multiplexer, it is also possible to employ more than one multiplexer by way of control. The same obviously goes for the embodiment of figure 1.

A central pin 52, to the relatively proximal end of which the relatively proximal end of the balloon 51 has been arranged, extends through the basic body 59 of the catheter. After deflating the balloon 51, it can be pulled back inside the basic body 59, by moving the pin 52 in a longitudinal direction towards the proximal end. The state in which the balloon 51 has been received inside the basic body 59 has been illustrated in figure 10.

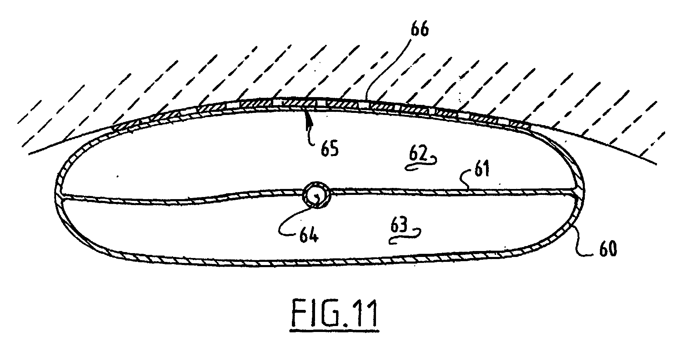

Finally, figure 11 shows a possible cross-section of a catheter according to the invention whereby the carrier comprises a balloon 60. The balloon has been preformed in such a manner that it can have the elongated shape of the cross-section shown in the figure. At a top surface , illustrated in figure 11, an electrode array 65 has been arranged. A partition 61 extends transversely through the balloon 60 as a result of which two compartments 62, 63 are formed. The compartments 62, 63 can be filled in different manners. The compartment 63 can for instance be inflated harder, forming a firm base, whereas the compartment 62 can be inflated relatively lightly as a result of which the support of the electrode array 65 is resilient and this electrode array 65 can adjust properly to the surface 66 to be treated. With this embodiment a central pin 64 has been drawn as well and serves for pulling the balloon into the basic body.

## Claims

1. Catheter (1) comprising a tube-like basic body (2) with a proximal end and a distal end, a connecting member arranged at the proximal end and an electrode array carried at the distal end, wherein the electrode array (5) is plate-like and has been arranged on a pliable carrier (18), which is movable between a folded state and an unfolded state transversely to the catheter, wherein folding means have been arranged for moving the carrier between the folded state and the unfolded state and furthermore comprising signal lines connected to the electrode array and extending to the proximal end, **characterized in that** the carrier comprises a sheet of memory metal having a transition temperature above body temperature, of which the folded state is a relatively relaxed state below the transition temperature and the unfolded state a relatively relaxed state at a temperature above the transition temperature and **in that** the carrier (18) is provided with heating means which are connected to a supply line (21, 22) extending to the proximal end, and are capable of heating the sheet to above the transition temperature.

2. Catheter as claimed in claim 1, wherein the basic body comprises an outer tube-like body (3) with a central lumen, which has a bore which is at least marginally larger than the cross-section of the electrode array (5) in folded state and the carrier (18) is movable between a retracted position in folded state inside the central lumen and a position extended from there, and wherein retraction means have been arranged for the purpose of moving the carrier (18) between the retracted and the extended position.

3. Catheter as claimed in claim 2, wherein the basic body (2) comprises an inner tube-like body movable inside the central lumen of the outer tube-like body (4) and the carrier (18) has been arranged at the distal end of the inner tube-like body (4).

4. Catheter as claimed in claim 2, wherein the carrier is elastically deformable and is relatively relaxed in the unfolded state and wherein guiding means have been arranged at the distal end of the outer tube-like body (3) for the purpose of moving the carrier (18) from the unfolded into the folded state during the change from the extended into the retracted position.

5. Catheter as claimed in one of the previous claims, wherein the electrode array (5) and the carrier (18) are rolled up around a longitudinal axis in the folded state.

6. Catheter as claimed in one of the preceding claims, wherein the heating means are made up of at least two separate sections, separated transversely.

7. Catheter as claimed in one of the previous claims, wherein a resilient, compressible cushion has been arranged in between the electrode array and the carrier.

8. Catheter as claimed in claim 7, wherein the resilient, compressible cushion is a bag to be filled with a fluid.

9. Catheter as claimed in claim 1, wherein the transition temperature of the memory metal is about 45'C.

10. Catheter as claimed in claim 1, wherein the carrier comprises an inflatable balloon (51).

11. Catheter as claimed in claim 10, wherein the balloon is connected with a distal end to an elongated body extending through the basic body in a movable manner, which can be used to pull the balloon into or extend it from the basic body.

12. Catheter as claimed in claim 10 or 11, wherein the balloon comprises a partition (61) running parallel to a wall carrying the electrode array (65) and the compartments formed by this partition (61) can be supplied with fluid under pressure via separate lines (58) extending to the proximal end.

13. Catheter as claimed in claim 12, wherein the compartment (63) turned away from the electrode array (65) is filled with a liquid.

14. Catheter as claimed in one of the previous claims, wherein at least one multiplexer (9) has been received in the basic body (2) close to the distal end, which is connected with each of the electrodes (8) of the electrode array (18) on one side, and to the signal lines on the other side.

15. Catheter as claimed in one of the previous claims, wherein the electrode array comprises a foil substrate (43), and connecting lines of the electrodes with the multiplexer have been made in the form of printed wiring on the foil substrate (43).

16. Catheter as claimed in claim 15, wherein the electrodes have been formed as printed wiring on the foil substrate (43) on the other side from the connecting lines and the connecting lines are connected with the electrodes via metallized openings in the foil substrate.

17. Catheter as claimed in one of the claims 14 - 16 inclusive, wherein the multiplexer (57) has been arranged on the foil substrate (43).

18. Catheter as claimed in one of the previous claims, wherein the carrier is connected with a rigid support connected to the basic body in a fixed manner, extending in the longitudinal direction thereof.

## Patentansprüche

1. Katheter (1), aufweisend einen rohrartigen Basiskörper (2) mit einem proximalen Ende und einem distalen Ende, ein Verbindungsteil, das an dem proximalen Ende angeordnet ist, und ein Elektrodenfeld, das an dem distalen Ende getragen wird, wobei das Elektrodenfeld (5) plattenartig ist und an einem biegsamen Träger (18) angeordnet wurde, welcher zwischen einem gefalteten Zustand und einem entfalteten Zustand quer zu dem Katheter bewegbar ist, wobei Faltmittel zum Bewegen des Trägers zwischen dem gefalteten Zustand und dem entfalteten Zustand angeordnet wurden, und ferner aufweisend Signalleitungen, die mit dem Elektrodenfeld verbunden sind und sich zu dem proximalen Ende erstrecken, **dadurch gekennzeichnet, dass** der Träger eine Folie aus Memory-Metall mit einer Übergangstemperatur oberhalb der Körpertemperatur aufweist, von welcher der gefaltete Zustand ein relativ entspannter Zustand unterhalb der Übergangstemperatur und der entfaltete Zustand ein relativ entspannter Zustand mit einer Temperatur oberhalb der Übergangstemperatur ist, und dass der Träger (18) mit Heizmitteln versehen ist, welche mit einer Versorgungsleitung (21, 22), die sich zu dem proximalen Ende erstreckt, verbunden sind und zum Erwärmen der Folie auf mehr als die Übergangstemperatur geeignet sind.

2. Katheter nach Anspruch 1, wobei der Basiskörper einen äußeren rohrartigen Körper (3) mit einem mittleren Lumen aufweist, welcher eine Bohrung hat, welche zumindest am Rande größer als der Querschnitt des Elektrodenfeldes (5) im gefalteten Zustand ist, und der Träger (18) zwischen einer zurückgezogenen Position im gefalteten Zustand innerhalb des mittleren Lumens und einer davon ausgestreckten Position bewegbar ist, und wobei die Zurückziehmittel für den Zweck der Bewegung des Trägers (18) zwischen der zurückgezogenen und der ausgestreckten Position angeordnet wurden.

3. Katheter nach Anspruch 2, wobei der Basiskörper (2) einen inneren rohrartigen Körper aufweist, der innerhalb des mittleren Lumens des äußeren rohrartigen Körpers (4) bewegbar ist, und der Träger (18) an dem distalen Ende des inneren rohrartigen Körpers (4) angeordnet wurde.

4. Katheter nach Anspruch 2, wobei der Träger elastisch verformbar ist und im entfalteten Zustand relativ entspannt ist, und wobei Führungsmittel an dem distalen Ende des äußeren rohrartigen Körpers (3) für den Zweck der Bewegung des Trägers (18) aus dem entfalteten in den gefalteten Zustand während der Änderung von der ausgestreckten in die zurückgezogene Position angeordnet wurde.

5. Katheter nach einem der vorhergehenden Ansprüche, wobei das Elektodenfeld (5) und der Träger (18) im gefalteten Zustand um eine Längsachse aufgerollt sind.

6. Katheter nach einem der vorhergehenden Ansprüche, wobei die Heizmittel aus zumindest zwei separaten Abschnitten aufgebaut sind, die quer getrennt sind.

7. Katheter nach einem der vorhergehenden Ansprüche, wobei ein federnd zusammendrückbares Kissen zwischen dem Elektrodenfeld und dem Träger angeordnet wurde.

8. Katheter nach Anspruch 7, wobei das federnd zusammendrückbare Kissen ein mit einer Flüssigkeit zu füllender Beutel ist.

9. Katheter nach Anspruch 1, wobei die Übergangstemperatur des Memory-Metalls etwa 45°C ist.

10. Katheter nach Anspruch 1, wobei der Träger einen aufblasbaren Ballon (51) aufweist.

11. Katheter nach Anspruch 10, wobei der Ballon an einem distalen Ende mit einem langgestreckten Körper verbunden ist, der sich durch den Basiskörper hindurch in einer beweglichen Weise erstreckt, welcher zum Ziehen des Ballons in den Basiskörper hinein oder zu dessen Ausstrecken aus diesem heraus verwendet werden kann.

12. Katheter nach Anspruch 10 oder 11, wobei der Ballon eine Trennwand (61) aufweist, die parallel zu einer Wand verläuft, die das Elektrodenfeld (65) trägt, und die Kammern, die von dieser Trennwand (61) gebildet werden, über separate Leitungen (58), die sich zu dem proximalen Ende erstrecken, mit Flüssigkeit unter Druck versorgt werden können.

13. Katheter nach Anspruch 12, wobei die Kammer (63), die von dem Elektrodenfeld (65) abgewandt ist, mit einer Flüssigkeit gefüllt ist.

14. Katheter nach einem der vorhergehenden Ansprüche, wobei zumindest ein Multiplexer (9) in dem Basiskörper (2) in der Nähe des distalen Endes aufgenommen wurde, welcher an der einen Seite mit jeder der Elektroden (8) des Elektrodenfeldes (18) und an der anderen Seite mit den Signalleitungen verbunden ist.

15. Katheter nach einem der vorhergehenden Ansprüche, wobei das Elektrodenfeld ein Foliensubstrat (43) aufweist, und Verbindungsleitungen der Elektroden mit dem Multiplexer in der Form einer gedruckten Schaltung an dem Foliensubstrat (43) gebildet wurden.

16. Katheter nach Anspruch 15, wobei die Elektroden als gedruckte Schaltung an dem Foliensubstrat (43) an der anderen Seite von den Verbindungsleitungen geformt wurden und die Verbindungsleitungen über metallisierte Öffnungen in dem Foliensubstrat mit den Elektroden verbunden sind.

17. Katheter nach einem der Ansprüche 14-16 umfassend, wobei der Multiplexer (57) an dem Foliensubstrat (43) angeordnet wurde.

18. Katheter nach einem der vorhergehenden Ansprüche, wobei der Träger mit einer festen Stütze verbunden ist, die mit dem Basiskörper in einer feststehenden Weise in der Längsrichtung davon sich erstreckend verbunden ist.

## Revendications

1. Cathéter (1) comprenant un corps de base semblable à un tube (2) comportant une extrémité proximale et une extrémité distale, un élément de raccordement agencé au niveau de l'extrémité proximale et un réseau d'électrodes supporté au niveau de l'extrémité distale, dans lequel le réseau d'électrodes (5) est semblable à une plaque et a été agencé sur un support pliable (18), qui est mobile entre un état plié et un état déployé transversalement au cathéter, dans lequel des moyens de pliage ont été agencés pour déplacer le support entre l'état plié et l'état déployé et comprenant, en outre, des lignes de signaux connectées au réseau d'électrodes et s'étendant jusqu'à l'extrémité proximale, **caractérisé en ce que** le support comprend une feuille de métal à déformation réversible ayant une température de transition supérieure à la température corporelle, dont l'état plié est un état relativement relaxé au-dessous de la température de transition et l'état déployé est un état relativement relaxé à une température supérieure à la température de transition, et **en ce que** le support (18) est pourvu de moyens de chauffage qui sont connectés à une ligne d'alimentation (21, 22) s'étendant jusqu'à l'extrémité proximale et qui sont capables de chauffer la feuille à une température supérieure à la température de transition.

2. Cathéter selon la revendication 1, dans lequel le corps de base comprend un corps extérieur semblable à un tube (3) comportant un lumen central, qui comporte un alésage qui est au moins légèrement plus grand que la section transversale du réseau d'électrodes (5) dans l'état plié, et le support (18) est mobile entre une position de rétraction dans l'état plié à l'intérieur du lumen central et une position d'extension à partir de celui-ci, et dans lequel des moyens de rétraction ont été agencés en vue du déplacement du support (18) entre les positions de rétraction et d'extension.

3. Cathéter selon la revendication 2, dans lequel le corps de base (2) comprend un corps intérieur semblable à un tube mobile à l'intérieur du lumen central du corps extérieur semblable à un tube (4) et le support (18) a été agencé au niveau de l'extrémité distale du corps intérieur semblable à un tube (4).

4. Cathéter selon la revendication 2, dans lequel le support est déformable élastiquement et est relativement relaxé dans l'état déployé, et dans lequel des moyens de guidage ont été agencés au niveau de l'extrémité distale du corps extérieur semblable à un tube (3) en vue du déplacement du support (18) de l'état déployé dans l'état plié pendant le passage de la position d'extension dans la position de rétraction.

5. Cathéter selon l'une des revendications précédentes, dans lequel le réseau d'électrodes (5) et le support (18) sont enroulés autour d'un axe longitudinal dans l'état plié.

6. Cathéter selon l'une des revendications précédentes, dans lequel les moyens de chauffage sont composés d'au moins deux sections séparées, séparées transversalement.

7. Cathéter selon l'une des revendications précédentes, dans lequel un coussin élastique, compressible, a été agencé entre le réseau d'électrodes et le support.

8. Cathéter selon la revendication 7, dans lequel le coussin élastique, compressible, est un sac destiné à être rempli d'un fluide.

9. Cathéter selon la revendication 1, dans lequel la température de transition du métal à déformation réversible est d'environ 45°C.

10. Cathéter selon la revendication 1, dans lequel le support comprend un ballonnet gonflable (21).

11. Cathéter selon la revendication 10, dans lequel le ballonnet est relié, au niveau d'une extrémité distale, à un corps allongé s'étendant à travers le corps de base de manière mobile, qui peut être utilisé pour pousser le ballonnet dans le corps de base ou le sortir de celui-ci.

12. Cathéter selon la revendication 10 ou 11, dans lequel le ballonnet comprend une séparation (61) s'étendant parallèlement à une paroi supportant le réseau d'électrodes (65) et les compartiments formés par cette séparation (61) peuvent recevoir un fluide sous pression par l'intermédiaire de conduites séparées (58) s'étendant jusqu'à l'extrémité proximale.

13. Cathéter selon la revendication 12, dans lequel le compartiment (63) orienté à l'opposé du réseau d'électrodes (65) est rempli d'un liquide.

14. Cathéter selon l'une des revendications précédentes, dans lequel au moins un multiplexeur (9) a été reçu dans le corps de base (2) à proximité de l'extrémité distale, qui est connecté à chacune des électrodes (8) du réseau d'électrodes (18) d'un côté, et aux lignes de signaux de l'autre côté.

15. Cathéter selon l'une des revendications précédentes, dans lequel le réseau d'électrodes comprend un substrat en feuille (43), et les lignes de connexion des électrodes au multiplexeur ont été réalisées sous la forme d'un câblage imprimé sur le substrat en feuille (43).

16. Cathéter selon la revendication 15, dans lequel les électrodes ont été formées en tant que câblage imprimé sur le substrat en feuille (43) sur l'autre côté par rapport aux lignes de connexion et les lignes de connexion sont connectées aux électrodes par l'intermédiaire d'ouvertures métallisées dans le substrat en feuille.

17. Cathéter selon l'une des revendications 14 à 16 incluses, dans lequel le multiplexeur (57) a été agencé sur le substrat en feuille (43).

18. Cathéter selon l'une des revendications précédentes, dans lequel le support est relié à un support rigide relié au corps de base de manière fixe, s'étendant dans la direction longitudinale de celui-ci.
